# EUROPEAN PATENT APPLICATION

(11) **EP 1 731 521 A1**
(43) Date of publication of application: **13.12.2006**
(21) Application number: 05728435.8
(22) Date of filing: 30.03.2005
(51) Int. Cl.: C07D 493/04, A23L 1/30, A61K 31/36, A61P 3/04, A61P 3/10, A61P 9/00, A61P 43/00

(54) **ADIPONECTIN INCREASING AGENT**

(30) Priority: 31.03.2004 JP 2004106289; 28.06.2004 JP 2004189719
(71) Applicant: SUNTORY LIMITED, Osaka-shi, Osaka 530-8203 (JP)
(72) Inventor: ONO, Yoshiko;, Osaka-shi, Osaka 5330022 (JP); FUJIWARA, Yoko, 1080073 (JP)
(74) Representative: Stoner, Gerard Patrick
(86) International application number: PCT/JP2005/006733
(87) International publication number: WO 2005/095414

(57) **Abstract**

An adiponectin enhancer comprising as an active ingredient sesamin and/or episesamin, or an analogue thereof.

## Description

### Technical Field of the Invention

The present invention relates to a composition containing an adiponectin enhancer comprising as an active ingredient sesamin and/or episesamin, or an analogue thereof.

### Background Art

Adipose tissue was long considered to play the single role of an energy storage reservoir, by storing energy and supplying it when needed. Recently, however, the importance of adipose tissue in metabolic disorders, and particularly lifestyle related diseases, has become the focus of much attention.

Obesity is a fundamental cause of lifestyle related diseases, and its major characteristic is enlargement of adipocytes. Since physiological adipocyte number increase by adipocyte differentiation almost never occurs after puberty, the increase in adipose tissue is believed to be due entirely to enlargement of individual adipocytes. Recent research has demonstrated that in addition to the function of adipocytes of storing excess energy in the form of neutral fats, adipose tissue also produces and secretes various adipocytokines such as adiponectin, leptin, tumor necrosis factor (TNFα), resistin, free fatty acids (FFA) and plasminogen activator (PAI-1), and that TNFα, resistin and FFA are produced and secreted in large amounts by enlarged adipocytes and provoke insulin resistance in skeletal muscle and the liver (Nichiyaku Rishi, 122, 317-324, 2003).

TNFα binds to TNFα receptor on adipocytes and hepatocytes and phosphorylates the serine of IRS-1 (insulin receptor substrate-1) via sphingomyelinase activity, resulting in attenuation of insulin function *(*Science, 259, 87-91, 1993).

On the other hand, adiponectin is also known as an adipose tissue-specific 30 kDa secreted protein which is highly expressed by adipocytes and constitutes 0.01% of human plasma protein *(*Biochem. Biophys. Res. Commun., 221, 286-289, 1996, 257, 79-83, 1999). Adiponectin plasma concentration in healthy volunteers is considered to be 1.9-17.0 mg/ml and the secretion of adiponectin, which is mainly from small adipocytes, decreases with progressing enlargement of adipocytes, resulting in reduced plasma concentration.

Specifically, in humans the plasma concentrations of PAI-1, TNFα and leptin are all increased with obesity and fat accumulation, while the plasma concentration of adiponectin is reduced in obese individuals but higher with decreasing body weight *(*Biochem. Biophys. Res. Commun., 257, 79-83, 1999). In particular, it has been learned that plasma concentration of adiponectin falls with accumulation of body fat, which is closely associated with obesity complications.

Reduction in plasma adiponectin concentration is a confirmed factor in coronary artery disease, independent from obesity index *(*Circulation, 100, 2473-2476, 1999). In diabetes as well, plasma adiponectin concentration is lower in proportion to severity of the diabetes (Arteriosclear. Thromb. Vasc. Biol., 20, 1595-1599, 2000), while the studies in rhesus monkeys have been reported which demonstrate that during the progression of obese type 2 diabetes caused by overeating and lack of exercise, hypoadiponectinemia precedes hyperinsulinemia, which is an index of insulin resistance, and diabetes (Diabetes, 50, 1126-1133, 2001)

In primary hypoadiponectinemia associated with human gene mutations of adiponectin, specifically in nine individuals with the 164th isoleucine mutated to threonine, the plasma adiponectin concentration was 25-30% of normal, with seven of the nine individuals exhibiting diabetes and two exhibiting borderline diabetes, while six of the nine cases were coronary artery disease patients who had experienced myocardial infarction, angina pectoris or the like. Primary hypoadiponectinemia is also associated with insulin resistance and coronary artery disease *(*Diabetes, 51, 2325-2328, 2002).

Cardiovascular complications often occur in renal failure patients, but the rate is reported to be significantly higher in patients with low plasma adiponectin concentration *(*Circulation, 102, 1296-1301, 2000). Analysis of adiponectin knockout mice has confirmed that they exhibit high insulin resistance diabetes upon short-term feeding of a high-fat, high-sucrose diet for 2 weeks, but that the high insulin resistance is improved to wild levels by adenovirus-mediated supplementation of plasma adiponectin *(*Nature Med. 8, 731-737, 2000).

Judging from reports that human type 2 diabetes patients have a three times greater risk of myocardial infarction as healthy persons *(*Am. J. Med. 105(1A), 4S-14S, 1998), it is gradually becoming undeniable that reduced plasma adiponectin concentration which leads to diabetes also promotes onset of myocardial infarction *(*Tanpakushitsu Kakusan Kouso, 47(14), 1896-1903, 2003).

Thus, research has indicated the importance of reducing adipocyte size and suppressing accumulation of enlarged adipocytes in order to promote production and secretion of adiponectin for increased plasma adiponectin, and suppressing secretion of cytokines with insulin resistance-eliciting effects, such as TNFα, from the standpoint of preventing or ameliorating lifestyle related diseases. In other words, it is now thought that by inducing differentiation of small adipocytes and suppressing accumulation of enlarged adipocytes in order to inhibit production of TNFα secreted by enlarged adipocytes and augment adiponectin production to raise serum and/or plasma adiponectin concentration, it is possible to prevent not only insulin resistance but also heart disease including diabetic large artery disease and coronary artery disease, or at least improve the symptoms of such diseases.

Recently it has been demonstrated that thiazolidine derivatives, which are peroxisome proliferator-activated receptor γ (PPARγ) agonists, increase small adipocytes which have newly differentiated from differentiated adipocytes, while also inducing apoptosis of enlarged adipocytes which overproduce insulin resistance-eliciting factors, thereby reducing their number and improving insulin resistance (J. Clin. Invest., 101, 1354-1361, 1998). It has also been reported that thiazolidine derivatives raise plasma adiponectin concentrations in patients with impaired glucose tolerance (Diabetes, 50, 2094-2099, 2001). However, no report has described a naturally-derived, safe food, beverage or composition which induces and increases small adipocytes and raises serum and/or plasma adiponectin concentration.

Sesamin is a principal lignan compound found in sesame, at a content of about 0.1-1%. Sesamin is known to have a Δ5 desaturase inhibiting effect (S. Shimizu et al., J. Am. Oil Chem. Soc. 66, 237-241 (1989), S. Shimizu et al. Lipid, 26, 512 (1991)), an antioxidant effect (Japanese Unexamined Patent Publication HEI No. 05-051388 and Japanese Patent Publication HEI No. 11-327924), an anti-hypertensive effect (Japanese Unexamined Patent Publication HEI No. 08-268887), a liver function improving effect (Japanese Unexamined Patent Publication HEI No. 04-099331) and a cholesterol lowering effect (Japanese Unexamined Patent Publication HEI No. 04-159221). The cholesterol lowering effect is synergized in the presence of α-tocopherol (A. Nakabayashi et al. Internal J. Nutr. Res., 65, 162 (1995), Japanese Unexamined Patent Publication HEI No. 04-368326).

The methanol-soluble and butanol-soluble fractions extracted from delipidated sesame, consisting of sesamin and sesamol, have glucose and lipid metabolism activating effects (Japanese Unexamined Patent Publication HEI No. 11-246427). Also, evaluation of the promoting effect on differentiation from adipocyte precursors (fibroblasts) to adipocytes has indicated that promoting differentiation to adipocytes increases intracellular triglyceride (TG) levels and glycerol-3-phosphate dehydrogenase activity. Insulin-mediated glucose uptake has also been shown to increase in the presence of sesamin, for which it has been listed as a glucose/lipid metabolism activator. The examples provided in the aforementioned publication demonstrate that dexamethasone and insulin are used as promoters of adipocyte precursor differentiation.

However, dexamethasone and insulin have been shown to cause accumulation of enlarged adipocytes and reduce adiponectin secretion *(*Biochem. Biophys. Res. Commun., 290, 1084-1089, 2002). These results therefore have not suggested any influence of the methanol-soluble and butanol-soluble fractions extracted from delipidated sesame, consisting of sesamin and sesamol, on adipocyte size or on adipocytokine secretion by adipocytes. In other words, although sesamin has been shown to have an effect of inducing differentiation from adipocyte precursors to adipocytes, its effect of causing accumulation and increase of normal small adipocytes, characterized by augmented production of adiponectin, has been neither investigated nor reported.
Patent document 1: Japanese Unexamined Patent Publication HEI No. 05-051388
Patent document 2: Japanese Patent Application HEI No. 11-327924
Patent document 3: Japanese Unexamined Patent Publication HEI No. 08-268887
Patent document 4: Japanese Unexamined Patent Publication HEI No. 04-099331
Patent document 5: Japanese Unexamined Patent Publication HEI No. 04-159221
Patent document 6: Japanese Unexamined Patent Publication HEI No. 04-368326
Patent document 7: Japanese Unexamined Patent Publication HEI No. 11-246427
Non-patent document 1: Nichiyaku Rishi, 122, 317-324, 2003
Non-patent document 2: Science, 259, 87-91, 1993
Non-patent document 3: Biochem. Biophys. Res. Commun., 221, 286-289, 1996
Non-patent document 4: Biochem. Biophys. Res. Commun., 257, 79-83, 1999
Non-patent document 5: Circulation, 100, 2473-2476, 1999
Non-patent document 6: Arteriosclear. Thromb. Vasc. Biol., 20, 1595-1599, 2000
Non-patent document 7: Diabetes, 50, 1126-1133, 2001
Non-patent document 8: Diabetes, 51, 2325-2328, 2002
Non-patent document 9: Circulation, 102, 1296-1301, 2000
Non-patent document 10: Nature Med. 8, 731-737, 2000
Non-patent document 11: Am. J. Med., 105(1A) 4S-14S, 1998
Non-patent document 12: Tanpakushitsu Kakusan Kouso 47(14), 1896-1903, 2003
Non-patent document 13: J. Clin. Invest., 101, 1354-1361, 1998
Non-patent document 14: Diabetes, 50, 2094-2099, 2001
Non-patent document 15: S. Shimizu et al., J. Am. Oil Chem. Soc. 66, 237-241 (1989)
Non-patent document 16: S. Shimizu et al., Lipid, 26, 512 (1991)
Non-patent document 17: Nakabayasi et al., : Internal. J. Nutr. Res., 65, 162 (1995)
Non-patent document 18: Biochem. Biophys. Res. Commun., 290, 1084-1089, 2002

### DISCLOSURE OF THE INVENTION

It is an object of the present invention to provide a natural and safe composition capable of inducing differentiation of adipocytes to small adipocytes and suppressing accumulation of enlarged adipocytes, of suppressing production of TNFα, of augmenting production of adiponectin and of raising serum and/or plasma adiponectin concentration. It is thereby possible to induce differentiation of adipocytes to small adipocytes, suppress accumulation of enlarged adipocytes or suppress production of TNFα, and to augment production of adiponectin in order to raise serum and/or plasma adiponectin concentration, thus helping to prevent or ameliorate not only insulin resistance but also heart diseases including diabetic large artery disease and coronary artery disease, and to prevent or ameliorate diseases associated with serum and/or plasma adiponectin reduction.

Enlargement of adipocytes is known to increase production of TNFα which elicits insulin resistance, and to lower secretion of adiponectin, leading to increased insulin resistance and various lifestyle related diseases. It may be expected that inducing size reduction of adipocytes and suppressing accumulation of enlarged adipocytes would improve unbalances in adipocytokines and help to prevent or ameliorate insulin resistance or lifestyle related diseases. The present inventors therefore actively researched foods, food components and traditional pharmaceuticals capable of inducing size reduction of adipocytes, and as a result completed the present invention upon establishing that the sesame lignan sesamin and/or its analogues induce size reduction of adipocytes, suppressing accumulation of enlarged adipocytes, and further that sesamin and/or its analogues augment production of adiponectin, increase the serum and/or plasma adiponectin concentration, while suppressing production of TNFα.

Thus, the present invention provides a food, beverage or composition which comprises sesamin and/or an analogue thereof as an active ingredient and which induces size reduction of adipocytes and suppresses accumulation of enlarged adipocytes,_as well as a process for its production. The invention further provides an adiponectin enhancer which comprises sesamin and/or an analogue thereof as an active ingredient, or a food, beverage or composition which augments adiponectin, as well as a process for its production. The invention still further provides a composition which comprises sesamin and/or episesamin or an analogue thereof as an active ingredient, and which has an effect of suppressing accumulation of enlarged adipocytes which produce TNFα.

### Brief Description of the Drawings

Fig. 1 is a photograph showing the influence of sesamin on adipocyte size (increase in smaller adipocytes).
Fig. 2 is a bar graph showing the increasing effect of sesamin on adiponectin mRNA expression in adipocytes.
Fig. 3 is a bar graph showing the suppressing effect of sesamin on TNFα mRNA expression in adipocytes.
Fig. 4 is a bar graph showing the influence of sesamin on adiponectin concentration in cultured adipocytes.

### Best Mode for Carrying Out the Invention

The present invention will now be explained in greater detail.

Currently, enlarged adipocytes are considered to be a root cause of lifestyle related diseases and diabetes. Of the adipocytokines secreted by enlarged adipocytes, TNFα is known to elicit insulin resistance. On the other hand, insulin resistance is improved by adiponectin, a "good" adipocytokine secreted by small adipocytes. Thus, it is important to suppress enlargement of and reduce the sizes of adipocytes to maintain a normal adipocytokine secretion balance, and to increase serum and/or plasma adiponectin concentration in order to prevent insulin resistance and diseases including lifestyle related diseases.

The present inventors therefore examined the effect of sesamin on adipocyte size. We also investigated adiponectin production in adipocyte cultures by enzyme-linked immunosorbent assay, and measured cellular gene expression of adipocytokines by RT-PCR. Further, the influence on the serum and/or plasma adiponectin concentration was confirmed by use of Zucker fatty rat.

As a result, it was confirmed that addition of sesamin to pre-differentiated 3T3-L1 cell cultures induces size reduction of adipocytes, increases adiponectin mRNA levels and production of adiponectin and decreases TNFα mRNA expression. Further, it was found that by continuous ingestion of sesamin, increase of serum and/or plasma adiponectin concentration could attained, and the present invention was thereupon completed.

Thus, the present invention provides foods, beverages, health foods, pharmaceuticals and feeds which comprise sesamin and/or an analogue thereof as an active ingredient and which can prevent or ameliorate obesity and lifestyle related diseases which are fundamentally caused by accumulation of enlarged adipocytes, as well as a process for their production. The invention further provides adiponectin enhancers and foods, beverages, health foods, pharmaceuticals and feeds having an effect of augmenting adiponectin, as well as a process for their production. More specifically, it provides foods, beverages, health foods, pharmaceuticals and feeds which can prevent or ameliorate insulin resistance caused by TNFα secreted by enlarged adipocytes, which can prevent and ameliorate insulin resistance, diabetic large artery disease and coronary artery disease by inducing increased adiponectin secretion from small adipocytes, and which can prevent and ameliorate various diseases whose onsets are linked to reduced serum and/or plasma adiponectin concentration, as well as a process for their production.

The present invention is not limited to sesamin and may also be applied for sesamin analogues. According to the invention, sesamin and its analogues are, for example, the dioxabicyclo[3.3.0]octane derivatives described in Japanese Unexamined Patent Publication HEI No. 4-9331, and specific examples include sesamin, sesaminol, episesamin, episesaminol, sesamolin, 2-(3,4-methylenedioxyphenyl)-6-(3-methoxy-4-hydroxyphenyl)-3,7-dioxabicyclo[3.3.0]octane, 2,6-bis(3-methoxy-4-hydroxyphenyl)-3,7-dioxabicyclo[3.3.0]octane, 2-(3,4-methylenedioxyphenyl)-6-(3-methoxy-4-hydroxyphenoxy)-3,7-dioxabicyclo[3.3.0]octane, 2-(3,4-methylenedioxyphenyl)-6-(3,4-dihydroxyphenyl)-3,7-dioxabicyclo[3.3.0]octane, 2-(3-methoxy-4-hydroxyphenyl)-6-(3,4-dihydroxyphenyl)-3,7-dioxabicyclo[3.3.0]octane and 2,6-bis(3,4-dihydroxyphenyl)-3,7-dioxabicyclo[3.3.0]octane. There may also be utilized glucosides of sesamin and its analogues, as well as metabolites of sesamin and its analogues.

Sesamin and its analogues according to the invention may be obtained, for example, by the method described in Japanese Unexamined Patent Publication HEI No. 4-9331, and may be used as extracts or if necessary as purified products.

When the sesamin and/or its analogue according to the invention is to be used as a food, beverage, health food or feed, there are encompassed not only health foods and beverages comprising the sesamin and/or its analogue, but also food additives containing the sesamin and/or its analogue. For use as a health food or beverage, for example, the composition may be added to a dry food, supplement, refreshment drink, mineral water, alcoholic beverage or the like, although there is no limitation to these.

When the sesamin and/or its analogue according to the invention is to be used as a prophylactic or therapeutic agent, the formulation may be solid or liquid, and there may be mentioned powders, tablets, pills, capsules, suppositories, granules, internal use mixtures, suspensions, emulsions, lotions and the like. Pharmaceutically acceptable excipients may also be added to a formulation for the invention. As excipients there may be used diluents, aromas, stabilizers, suspension lubricants, binders, preservatives, tablet disintegrators and the like, either alone or in combinations.

The effective amount for sesamin and/or its analogue (the active ingredient) according to the invention to induce size reduction of adipocytes, augment adiponectin production or suppress TNFα production may be, for example, 0.2-500 mg, preferably 0.5-100 mg and more preferably 1-60 mg per day. The effective amount will differ depending on the causative factors, the type of condition, the characteristics, age and body weight of the patient, the severity of symptoms and the form of administration.

The sesamin and/or its analogue as the active ingredient of a food, beverage, food or pharmaceutical of the invention is preferably ingested for a continuous prolonged period, if possible, in order to induce size reduction of adipocytes, augment production of adiponectin or suppress production of TNFα. There is no limitation on the timing or manner of ingestion, and ingestion at various opportunities can exhibit an effect of inducing adipocyte size reduction and increased plasma adiponectin concentration due to augmented adiponectin production.

### EXAMPLES

Examples will now be explained in detail with the understanding that they are in no way limitative on the invention.

### Example 1. Effect of inducing size reduction of adipocytes and influence on adiponectin and TNFα gene expression

Mouse-derived 3T3-L1 cells were treated for 42 hours in culturing solution containing insulin, dexamethasone and isobutylxanthine, for differentiation to adipocytes. The culture solution was removed and immediately combined with sesamin-free culturing solution (control) or culturing solution containing sesamin at a final concentration of 50 µM (sesamin), and after culturing for 48 hours, the culture solution was exchanged and culturing was continued for 6 days. The cell culturing was carried out in an incubator controlled to 5% CO₂ gas-95% air at a temperature of 37°C. The cellular mRNA levels for adiponectin and TNFα were measured by quantitative RT-PCR using CYBR-Green (BMC Biotechnol. 3, 18, 2003).

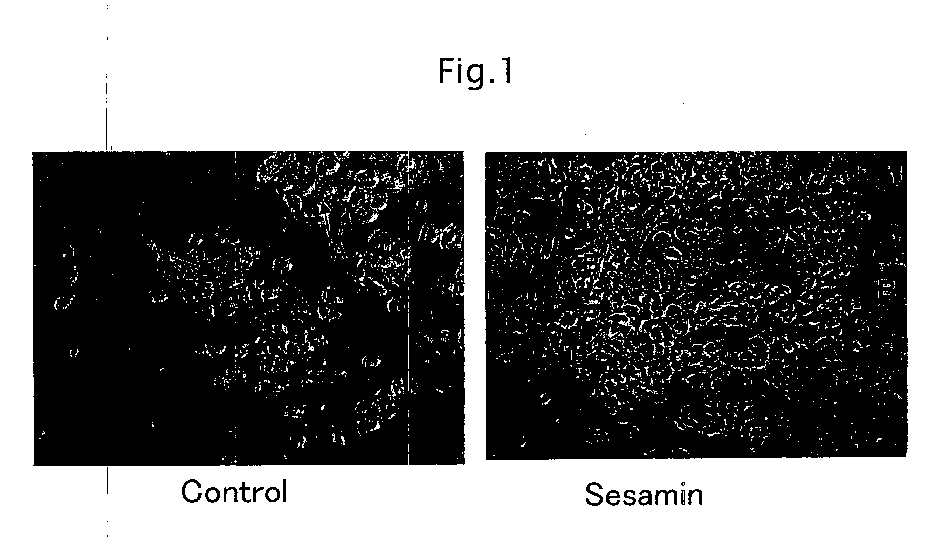

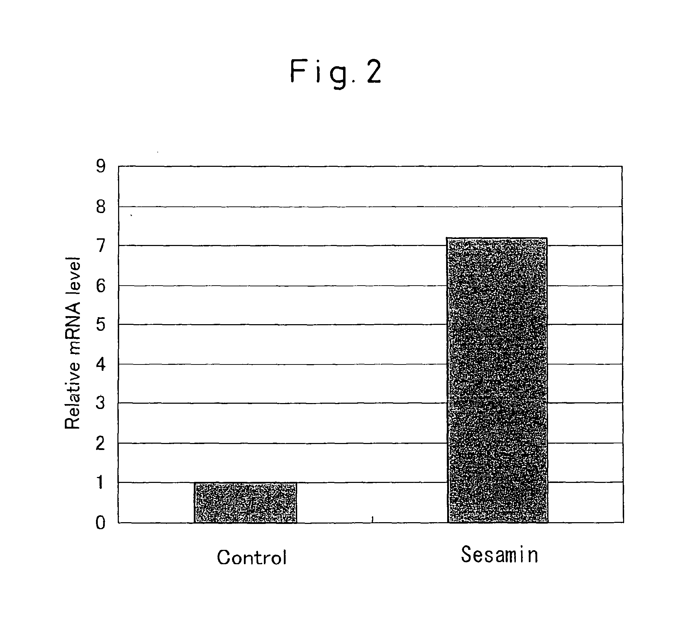

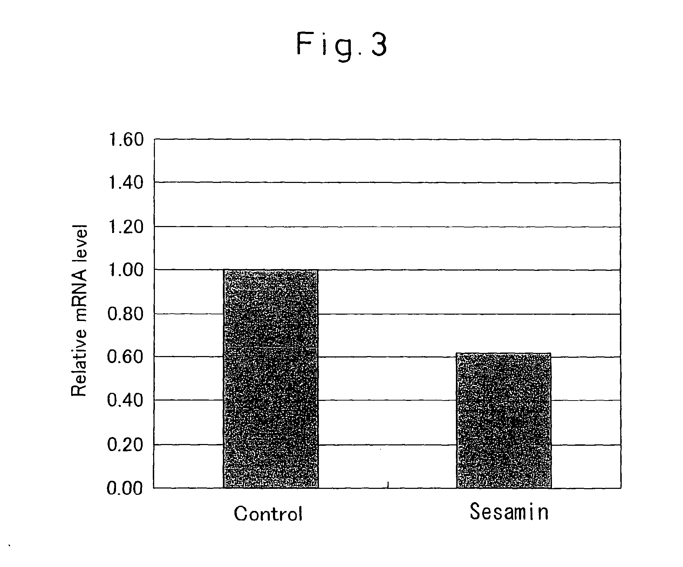

Results: Addition of sesamin to the 3T3-L1 cells which had been pre-differentiated to adipocytes, followed by 8 days of culturing, resulted in development of numerous small adipocytes (Fig. 1). Large-sized adipocytes were seen in the control group, as shown by arrow A. However, many small adipocytes were seen in the sesamin-added group (arrow B). The dark (red) sections represent accumulated fat, and several fat droplets were present in each cell in the control group, whereas no enlarged adipocytes containing numerous fat droplets in each cell were found in the sesamin treated group. The adiponectin mRNA levels in the adipocytes had increased 7-fold with respect to the control group (Fig. 2), while the TNFα mRNA levels were found to have decreased (Fig. 3).

These experimental results demonstrated that sesamin induced size reduction of adipocytes and suppressed accumulation of enlarged adipocytes. Adiponectin mRNA levels also increased while TNFα mRNA levels decreased.

### Example 2. Influence on adiponectin production by adipocytes

Mouse-derived 3T3-L1 cells were treated for 42 hours in culturing solution containing insulin, dexamethasone and isobutylxanthine, for differentiation to adipocytes. The culture solution was removed and immediately combined with sesamin-free culturing solution (control) or culturing solution containing sesamin at a final concentration of 50 µM (sesamin), and after culturing for 48 hours, the culture solution was exchanged and culturing was continued for 6 days. The cell culturing was carried out in an incubator controlled to 5% CO₂ gas-95% air at a temperature of 37°C. The adiponectin concentrations in the culture solutions were measured by Enzyme-Linked ImmunoSorbent Assay (Mouse/Rat Adiponectin ELISA Kit, Otsuka Pharmaceutical).

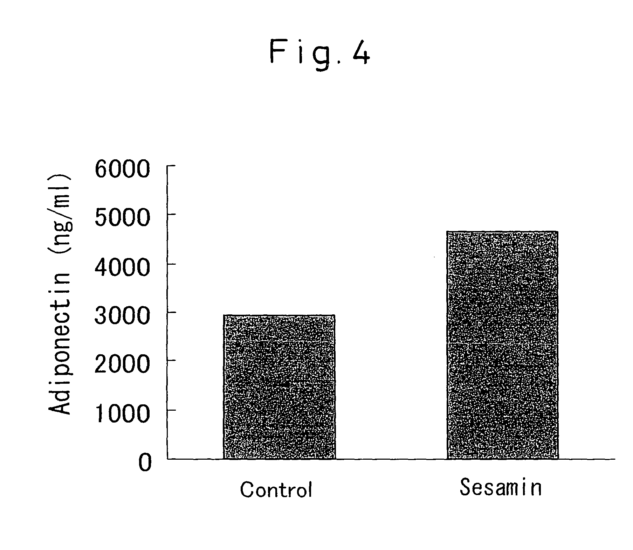

Results: Addition of sesamin to the 3T3-L1 cells which had been pre-differentiated to adipocytes increased adiponectin concentration in the cell cultured medium by 1.6-fold (Fig. 4).

### Example 3. Augmenting effect on plasma adiponectin conjugate

Five-week-old male Zucker fatty rats (crj; (ZUC)-fa/fa) were purchased from Charles River Japan and acclimatized to the test environment, and then animals exhibiting adequate development were supplied for the test. The rats were divided into two groups with five rats per group, with the first group being freely given AIN-93G (growth stage feed, product of Nihon Crea) as the control group and the second group being freely given feed comprising 0.1% sesamin added to AIN-93G, for a four week period. After four weeks, blood was sampled after overnight starving, and the plasma was separated. The plasma adiponectin concentrations were assayed using an Enzyme-Linked ImmunoSorbent Assay (Mouse/Rat Adiponectin ELISA Kit, product of Otsuka Pharmaceutical).

As a result, the plasma adiponectin concentrations of the rats given the sesamin-added feed for 1 month was 6.53 µg/ml, which was clearly higher than the rats given the control feed (6.06 µg/ml).

This result indicated that sesamin induces size reduction of adipocytes and inhibits accumulation of enlarged adipocytes, thereby increasing adiponectin production and secretion and increasing plasma adiponectin concentration.

**Table 1. Plasma adiponectin concentrations of Zucker fatty rats**

| Group | Plasma adiponectin (µg/ml) |
|---|---|
| Control feed | 6.06 ±0.48 |
| 0.1% sesamin feed | 6.53 ±0.54 |

This experimental result demonstrated that sesamin induced size reduction of adipocytes and suppressed accumulation of enlarged adipocytes, thus increasing production and secretion of adiponectin.

| Formulation Example | 1 Butter |
|---|---|
| Sesamin | 1.2 g |
| Butter fat | 100 g |
| Tocopherol acetate | 1.2 g |

After adding 1.2 g of sesamin to 100 g of butter fat from which the butter milk had been removed by churning in a butter making process, 1.2 g of tocopherol acetate was further added and the mixture was worked into an even texture to obtain butter containing a composition of the invention.

| Formulation Example 2 | Granules |
|---|---|
| Sesamin/episesamin mixture | 0.25 g |
| Tocopherol acetate | 0.25 g |
| Silicic anhydride | 20.5 g |
| Corn starch | 79 g |

The ingredients were uniformly mixed. After adding 100 ml of a 10% hydroxypropyl cellulose/ethanol solution to the mixture, it was kneaded by an ordinary method and extruded and dried to obtain granules.

| Formulation Example 3 | Tablets |
|---|---|
| Sesamin | 3.5 g |
| Tocopherol acetate | 0.5 g |
| Silicic anhydride | 20 g |
| Microcrystalline cellulose | 10 g |
| Magnesium stearate | 3 g |
| Lactose | 60 g |

The ingredients were combined and the mixture was formed into tablets using a single-shot tableting machine, to produce tablets with a diameter of 7 mm and a weight of 100 mg.

| Formulation Example 4 | Capsules |
|---|---|
| Gelatin | 70.0% |
| Glycerin | 22.9% |
| Methyl peroxybenzoate | 0.15% |
| Propyl peroxybenzoate | 0.51% |
| Water | q.s. |
| Total | 100% |

The composition shown below was filled into soft capsules comprising the components listed above by an ordinary method, to obtain soft capsules at 200 mg/capsule.

| | |
|---|---|
| Sesamin/episesamin mixture | 10.8 |
| Beeswax | 30 |
| α-Tocopherol | 20 |
| Palm oil | 10 |
| Wheat germ oil | q.s. |
| Total | 100% |

| Formulation Example 5 | Drink |
|---|---|
| Flavor: Sodium DL-tartrate | 0.1 g |
| Succinic acid | 0.009 g |
| Sweetness: Liquid sugar | 800 g |
| Acidity: Citric acid | 12 g |
| Vitamin: Vitamin C | 10 g |
| Sesamin | 1 g |
| Vitamin E | 30 g |
| Cyclodextrin | 5 g |
| Aroma | 15 ml |
| Potassium chloride | 1g |
| Magnesium sulfate | 0.5 g |

The components listed above were combined, and water was added to make 10 liters. The drink volume was approximately 100 ml per portion.

### Effect of the Invention

Ingestion of sesamin and/or an analogue thereof induces size reduction of adipocytes, suppresses accumulation of enlarged adipocytes, augments production of adiponectin and suppresses secretion of TNFα. Regulating secretion of adipocytokines by this effect of controlling adipocyte sizes is not only highly useful from the standpoint of preventing and alleviating lifestyle related diseases attributable to enlarged adipocytes, but is also highly useful for preventing or alleviating various diseases associated with reduced serum and/or plasma adiponectin concentration.

## Claims

1. An adiponectin enhancer comprising as an active ingredient sesamin and/or episesamin, or an analogue thereof.

2. A composition containing an adiponectin enhancer comprising as an active ingredient sesamin and/or episesamin, or an analogue thereof.

3. A composition with an inducing effect on small adipocytes, comprising as an active ingredient sesamin and/or episesamin, or an analogue thereof.

4. A composition with an effect of suppressing accumulation of TNFα-producing enlarged adipocytes, comprising as an active ingredient sesamin and/or episesamin, or an analogue thereof.

5. A method according to claim 2 for augmenting adiponectin by intake of sesamin and/or episesamin, or an analogue thereof.

6. A method according to claim 3 for promoting inducement of small adipocytes by intake of sesamin and/or episesamin, or an analogue thereof.

7. A method according to claim 4 for suppressing accumulation of TNFα-producing enlarged adipocytes by intake of sesamin and/or episesamin, or an analogue thereof.

8. A composition according to any one of claims 1 to 4, which is a food or drink.

9. A composition according to any one of claims 1 to 4, which is a pharmaceutical.

10. A method of augmenting adiponectin by administering sesamin and/or episesamin, or an analogue thereof.

11. A method of inducing small adipocytes by administering sesamin and/or episesamin, or an analogue thereof.

12. A method of suppressing accumulation of TNFα-producing enlarged adipocytes by administering sesamin and/or episesamin, or an analogue thereof.

13. A use of sesamin and/or episesamin, or an analogue thereof for production of a composition containing an adiponectin enhancer.

14. The use of sesamin and/or episesamin, or an analogue thereof for production of a composition with an inducing effect on small adipocytes.

15. The use of sesamin and/or episesamin, or an analogue thereof for production of a composition with an effect of suppressing accumulation of TNFα-producing enlarged adipocytes.

16. The use according to claim 2 for augmenting adiponectin by intake of sesamin and/or episesamin, or an analogue thereof.

17. The use according to claim 3 for promoting inducement of small adipocytes by intake of sesamin and/or episesamin, or an analogue thereof.

18. The use according to claim 4 for suppressing accumulation of TNFα-producing enlarged adipocytes by intake of sesamin and/or episesamin, or an analogue thereof.

19. The use according to any one of claims 1 to 4, which is a food or drink.

20. The use according to any one of claims 1 to 4, which is a pharmaceutical.
